(19) Europäisches Patentamt
European Patent Office
Office européen des brevets

(11) **EP 4 745 973 A1**

(12) **EUROPEAN PATENT APPLICATION**
published in accordance with Art. 153(4) EPC

(43) Date of publication:
**20.05.2026 Bulletin 2026/21**

(21) Application number: **24839217.7**

(22) Date of filing: **09.02.2024**

(51) International Patent Classification (IPC):
***G16B 30/00*** *(2019.01)* ***G16B 40/00*** *(2019.01)*

(52) Cooperative Patent Classification (CPC):
**G16B 30/00; G16B 40/00**

(86) International application number:
**PCT/JP2024/004447**

(87) International publication number:
**WO 2025/013319 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.07.2023 JP 2023113116**

(71) Applicant: **Hitachi, Ltd.**
**Tokyo 100-8280 (JP)**

(72) Inventor: **KIDO Kunihiko**
**Tokyo 100-8280 (JP)**

(74) Representative: **MERH-IP Matias Erny Reichl Hoffmann**
**Patentanwälte PartG mbB**
**Paul-Heyse-Straße 29**
**80336 München (DE)**

(54) **SEQUENCE INFORMATION PROCESSING DEVICE AND METHOD**

(57) An object of the present invention is to support efficient creation of various sequence information having constituent elements ensuring diversity. A configuration of the present invention is a sequence information processing device 1 that processes sequence information represented by a sequence of a plurality of elements, the sequence information processing device 1 including: an attention analysis processing unit 14 that identifies, based on a reward function that quantifies a characteristic of the sequence information, an inter-element pair of sequence information in which contribution of the reward function to output satisfies a predetermined condition; and a sampling function identification processing unit 15 that calculates a sampling function for creating the sequence information such that appearance frequencies of the plurality of elements are substantially equal in the identified inter-element pair.

*FIG. 3*

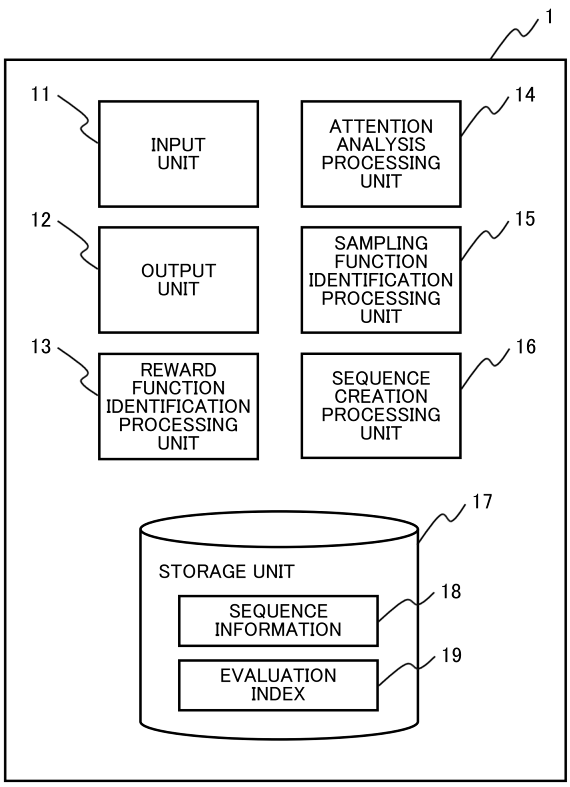


EP 4 745 973 A1

## Description

### Technical Field

The present invention relates to a technique for supporting creation of sequence information indicating sequences of various elements such as amino acids.

### Background Art

There is a need for a technique for efficiently creating sequence information indicating permutations and combinations of a large number of elements. For example, the design of antibody medicines requires multipurpose optimization for evaluating multifaceted characteristics such as affinity to an antigen, hydrophobicity, solubility, and immunogenicity based on a combination of amino acid sequences. An enormous amount of calculation is required to find a sequence that simultaneously satisfies such multifaceted characteristics. For example, when the number of sequences is 10 and the number of amino acids is 20, the total number of combinations is $20^{10}$ ($>10^{13}$), and a brute-force solution is not realistic.

Therefore, PTL 1 discloses a technique for "automatically creating a large number of mutated sequences in which a part of the amino acid sequence of a prototype antibody is mutated". In PTL 1, "a process of acquiring physical properties of a prototype antibody and an activity when the prototype antibody is combined with a predetermined target antigen as teacher data, and generating a learning model using the acquired physical properties as input data and the acquired activity as output data is executed by a computer. An information processing method includes causing a computer to execute processing of acquiring an amino acid sequence of a mutated antibody different from the prototype antibody, acquiring physical properties of the mutated antibody based on the amino acid sequence of the acquired mutated antibody, inputting the acquired physical properties of the mutated antibody to the learning model, and outputting an activity of the mutated antibody".

### Citation List

#### Patent Literature

PTL 1: JP 2020-077206 A

### Summary of Invention

#### Technical Problem

As described above, in Patent Literature 1, a prediction model is constructed from experimental data of physical properties and activities for mutated sequences, and candidates are extracted by applying reinforcement learning by highly active sequences and docking simulation. Herein, in the background art including Patent Literature 1, characters are sequentially selected by Bayesian optimization or the like using sequence information as character string data. The sequence information is represented by a reward function indicating a characteristic as the sequence information, and the sequence information created by this reward function is evaluated. In addition, learning a sampling function proportional to a reward function based on a flow matching condition creates sequence information for sequentially selecting characters based on the sampling function.

Herein, for the reward function, there is a method of constructing a prediction model from measurement data by machine learning or the like. In this case, data deviating from the measurement data deteriorates in accuracy. In addition, although a configuration of a reward function based on a theoretical mechanism of biology and chemistry is conceivable, complicated biological phenomena are simplified, and accuracy may be insufficient. Due to the insufficient performance of the reward function, it is often impossible to satisfy multifaceted characteristics when an actual experiment is performed on the created sequence. When only similar sequence information is created, in actual experiments and demonstrations, the majority of characteristics may not be appropriate and may not be available (for example, completely extinguished). In order to avoid this, it is desirable to create as diverse sequence information as possible while satisfying physical properties based on a reward function and conditions as active medical agents.

For example, it is required to create sequence information all over the sequence space without bias.

Therefore, an object of the present invention is to support efficient creation of various sequence information while satisfying multifaceted characteristics based on a reward function.

## Solution to Problem

In order to solve the above problems, in the present invention, in order to ensure diversity of sequence information, an inter-element pair of sequences in which contribution to output of a reward function indicating a characteristic of a sequence indicated by the sequence information satisfies a predetermined condition is identified. More specifically, there is provided a sequence information processing device that processes sequence information represented by a sequence of a plurality of elements, the sequence information processing device including: an attention analysis processing unit that identifies, based on a reward function that quantifies a characteristic of the sequence information, an inter-element pair of sequence information in which contribution of the reward function to output satisfies a predetermined condition; and a sampling function identification processing unit that calculates a sampling function for creating the sequence information such that appearance frequencies of the plurality of elements are substantially equal in the identified inter-element pair.

In addition, the present invention also includes a sequence information processing device that processes sequence information represented by a sequence of a plurality of elements, the sequence information processing device including: an input unit that receives the plurality of elements as an input; an attention analysis processing unit that identifies an inter-element pair of sequence information in which contribution of a reward function to output satisfies a predetermined condition based on the reward function that quantifies a characteristic of the sequence information; a sampling function identification processing unit that calculates a sampling function for creating the sequence information such that appearance frequencies of the plurality of elements are substantially equal in the identified inter-element pair; and an output unit that outputs the created sequence information.

In addition, the present invention also includes a sequence information processing system including at least a sequence information processing device. In addition, the sequence information processing method executed by the sequence information writing device and the sequence information processing system is also included in the present invention.

Further, a program for causing the above-described sequence information processing device to function as a computer and a storage medium storing the program are also included in the present invention.

## Advantageous Effects of Invention

The present invention can support efficient creation of various sequence information having constitute elements ensuring diversity.

## Brief Description of Drawings

[FIG. 1] FIG. 1 is a diagram illustrating amino acids in one embodiment of the present invention.
[FIG. 2] FIG. 2 is a system configuration diagram illustrating a sequence information processing system according to one embodiment of the present invention.
[FIG. 3] FIG. 3 is a functional block diagram of a sequence information processing device 1 according to one embodiment of the present invention.
[FIG. 4] FIG. 4 is a hardware configuration diagram of a sequence information processing device 1 according to one embodiment of the present invention.
[FIG. 5] FIG. 5 is a diagram illustrating sequence information 18 used in one embodiment of the present invention.
[FIG. 6] FIG. 6 is a diagram for explaining the concept of one embodiment of the present invention.
[FIG. 7] FIG. 7 is a flowchart illustrating a processing flow in one embodiment of the present invention.
[FIG. 8] FIG. 8 is a diagram for explaining attention in one embodiment of the present invention.
[FIG. 9] FIG. 9 is a diagram for explaining the degree of influence in one embodiment of the present invention.
[FIG. 10] FIG. 10 is a flowchart illustrating sampling function of a learning processing in step S7 according to one embodiment of the present invention.
[FIG. 11] FIG. 11 is a diagram schematically illustrating strength of contribution of adjacent pairs in one embodiment of the present invention.
[FIG. 12] FIG. 12 is a diagram schematically illustrating an evaluation index p of an adjacent pair according to one embodiment of the present invention.

## Description of Embodiments

Hereinafter, an embodiment of the present invention will be described with reference to the drawings. In the present embodiment, antibody medicines represented by an amino acid sequence having an amino acid as an element will be described as an example.

FIG. 1 is a diagram illustrating amino acids in the present embodiment. In FIG. 1, a symbol is assigned to each amino acid (name). The sequence of this symbol (for example, the sequence X = APRKNVRWCTISQPEWFKCR) indicates the ingredient of the antibody medical product to be developed. Then, it is verified whether the antibody medical product to be developed exhibits a desired efficacy that is an example of characteristics. In addition, in the present embodiment, an example of supporting development of antibody medicines in a plurality of pharmaceutical companies will be described. Note that the symbol is an example of an element, and can also be expressed as a character (character string/character pattern) or the like.

FIG. 2 is a system configuration diagram illustrating a sequence information processing system according to the present embodiment. In FIG. 2, the sequence information processing system is implemented by a cloud system that provides information services to a plurality of pharmaceutical companies. Specifically, in FIG. 2, each of the pharmaceutical companies 2a to 2c is connected to the sequence information processing device 1 implemented by a server via the network 3. In order to implement this, the pharmaceutical companies 2a to 2c have an in-house system having terminal devices 20a to 20c implemented by a PC or the like, and this is connected to the network 3.

In addition, the terminal devices 20a to 20c are connected to each other via an in-house network such as an intranet. Then, the terminal devices 20a to 20c notify the sequence information processing device 1 of various instructions and receive processing results from the sequence information processing device 1. Therefore, each of the terminal devices 20a to 20c includes a display device, an input device, a processing device, and a storage device, and the processing device executes various processes according to a program. Note that, in the present embodiment, the sequence information processing device 1 is implemented by an external server, but may be implemented as an in-house system like the sequence information processing device 1c in FIG. 2. Further, the cloud system in the present embodiment is one of implementation examples, and the sequence information processing device 1 may be implemented by a PC in a so-called stand-alone manner.

Next, a configuration of the sequence information processing device 1 that executes main processing in the present embodiment will be described. FIG. 3 is a functional block diagram of a sequence information processing device 1 according to the present embodiment. In FIG. 3, the sequence information processing device 1 includes an input unit 11, an output unit 12, a reward function identification processing unit 13, an attention analysis processing unit 14, a sampling function identification processing unit 15, a sequence creation processing unit 16, and a storage unit 17.

Herein, the input unit 11 receives information, instructions, and the like from the terminal devices 20a to 20c, the user, and the like. The input unit 11 is, for example, a keyboard, a mouse, a touch panel, a numeric keypad, a scanner, a microphone, or the like. In addition, the output unit 12 outputs information, a processing result, and the like, for example, created sequence information, to the terminal devices 20a to 20c, the user, and the like. The output unit 12 is, for example, a display, a printer, a speaker, or the like. In addition, the reward function identification processing unit 13 learns a prediction model for prediction, and identifies, that is, specifies the prediction model as a reward function (step S1). Herein, the reward function quantifies a characteristic of the sequence information.

In addition, the attention analysis processing unit 14 aggregates a relationship (attention) between amino acids that are elements in the target sequence information, and identifies an adjacent pair (combination) including adjacent elements, in which contribution to the reward function satisfies a predetermined condition at each position in the sequence information (step S2). In the present embodiment, the fact that the contribution to the reward function is smaller (weaker) than a predetermined threshold value is used as the predetermined condition. Note that the adjacent pair is an example of an inter-element pair constituted by a set of elements among a plurality of elements constituting the sequence information. For this reason, inter-element pairs of elements at distant positions may be used.

In addition, the sampling function identification processing unit 15 identifies a sampling function for creating sequence information. Herein, the sampling function is proportional to the reward function identified by the reward function identification processing unit 13, and includes the reward function. Herein, the sampling function identification processing unit 15 identifies a sampling function that minimizes the loss function (loss function) using the adjacent pair identified by the attention analysis processing unit 14 (step S3). Herein, when "minimizing the loss function", it is desirable that the appearance frequencies of elements in the sequence information created by the sampling function be substantially equal. Herein, the appearance frequency being substantially equal means that the difference in the number of appearances and the frequency of each element in the sequence information is within a predetermined range.

In addition, the sequence creation processing unit 16 creates sequence information by using the sampling function identified by the sampling function identification processing unit 15. Note that the creation of the sequence information may be executed by another device or may be omitted in the sequence information processing device 1. In addition, the storage unit 17 stores the sequence information 18 and the evaluation index 19. Although not illustrated in FIG. 3, functions such as the reward function, the loss function, and the sampling function described above, and rules for executing the functions may be stored. The sequence information 18 and the evaluation index 19 will be described later.

Next, an implementation example of the sequence information processing device 1 will be described. FIG. 4 is a hardware configuration diagram of a sequence information processing device 1 according to the present embodiment. In FIG. 4, the sequence information processing device 1 includes a processing device 101, a communication device 102, a memory 103, and a sub-storage device 104, which are connected to each other via a communication path.

First, the processing device 101 can be implemented by a processor such as a CPU, and executes an operation according to a sequence information processing program 105 stored in the sub-storage device 104 described later. The sequence information processing program 105 will be described later.

In addition, the communication device 102 corresponds to the input unit 11 and the output unit 12 in FIG. 3, is connected to the network 3, and communicates with other devices. Note that, in FIG. 3, the sequence information processing device 1 may be provided with an input device or a display device as the input unit 11 and the output unit 12.

In addition, the memory 103 and the sub-storage device 104 correspond to a storage unit 17 in FIG. 3. Then, the sequence information processing program 105 stored in the sub-storage device 104 and information used for processing in the processing device 101 are expanded in the memory 103. In addition, the sub-storage device 104 can be implemented by a so-called storage. Then, the sub-storage device 104 stores the sequence information processing program 105, the sequence information 18, and the evaluation index 19. In addition, the sub-storage device 104 may be implemented by various storage media such as an external hard disk drive (HDD), a solid state drive (SSD), and a memory card, or may be implemented by a device different from the sequence information processing device 1 such as a file server.

Herein, the sequence information processing program 105 includes a reward function identification processing module 106, an attention analysis processing module 107, a sampling function identification processing module 108, and a sequence creation processing module 109. Each of these modules may be implemented by an individual program or a combination of some programs.

Herein, the configuration illustrated in FIG. 3 that executes the same function as each module is as follows.

Reward function identification processing unit 13: reward function identification processing module 106
Attention analysis processing unit 14: attention analysis processing module 107
Sampling function identification processing unit 15: sampling function identification processing module 108
Sequence creation processing unit 16: Sequence creation processing module 109
Therefore, the processing device 101 executes processing of the reward function identification processing unit 13, the attention analysis processing unit 14, the sampling function identification processing unit 15, and the sequence creation processing unit 16 in accordance with the sequence information processing program 105.

Note that at least some of the functions and processes of the sequence information processing device 1 may be provided in the terminal devices 20a to 20c.

Next, each piece of information used in the present embodiment and stored in the sub-storage device 104 will be described. First, FIG. 5 is a diagram illustrating sequence information 18 used in the present embodiment. The sequence information 18 is created by the sequence information processing device 1 and indicates an ingredient of an antibody medicine to be developed.

In FIG. 5, symbols 1 to N indicate amino acids as elements. For example, the sequence information of NO1 is the sequence shown in FIG. 1, and includes amino acids, A: alanine, P: proline, R: arginine, and K: lysine to R: arginine. In addition, in the sequence information of NO2, symbol 3 is changed to L: leucine as compared with NO1. In addition, in the present embodiment, it is assumed that M pieces of sequence information of NO1 to M are created.

In addition, the evaluation index 19 is information used as teacher data for evaluating the sequence. The description of the information in the present embodiment ends, and the processing in the present embodiment will be described below. Herein, first, the concept of the present embodiment will be described, and next, a processing flow will be described. FIG. 6 is a diagram for explaining the concept of the present embodiment. In FIG. 6, (1) the sequence x has elements combined in the order of $\cdots s' \rightarrow s \rightarrow s'' \cdots$. Then, the sampling function (F) satisfying the flow matching condition in the case of the order of each element is calculated. Herein, as the flow matching condition, it is desirable that inflow and outflow of elements are the same. However, it is significantly difficult or rare to always be "the same". Therefore, as shown in (Mathematical formula 1), a sampling function (F) that minimizes the loss function (L) is obtained as an approximate solution.

[Mathematical formula 1]

$$L(s \rightarrow s') = \left( log \frac{\sum_{s' \in Parent(s)} F(s' \rightarrow s)}{\sum_{s'' \in child(s)} F(s \rightarrow s'')} \right)^2 \quad \text{... (Mathematical formula 1)}$$

Then, for an adjacent pair including adjacent elements in which contribution of the reward function satisfies a predetermined condition (for example, it is weaker than the threshold), a loss function (L) as shown in (Mathematical formula 2) is identified.

[Mathematical formula 2]

$$L(s \to s') = a \sum_{s' \in child(s)} P(s \to s') \log P(s \to s') + b|F(s \to s')| \quad \text{... (Mathematical formula 2)}$$

Herein, the significance of the above processing will be described. First, in the present embodiment, it is aimed that the sequence of elements is randomized (leveled in sequence space). However, merely randomization results in a huge amount of calculation, and there is no probability that the reward can be increased. For this reason, a sampling function that minimizes a loss function is used as an approximate solution for randomization, that is, probability equalization with respect to "a portion of low importance" for characteristics such as efficacy. Herein, regarding the "unimportant portion ", it is desirable that the contribution of the reward function is weak, that is, the contribution is equal to or less than a threshold, but the contribution may satisfy a predetermined condition.

Then, in FIG. 6, (2) sampling of an adjacent pair in which the contribution of the reward function is weak is performed to implement a probability in which the appearance frequencies are substantially equal. As a result, the reward is greatly shown in the evaluation result in the function (closer to desirable efficacy and the like), and randomized various sequence information can be created.

Next, a processing flow for implementing the above idea will be described. FIG. 7 is a flowchart illustrating a processing flow in the present embodiment. Hereinafter, this flowchart will be described with reference to the configuration of FIG. 3.

First, in step S1, the reward function identification processing unit 13 acquires sequence information indicating the entire sequence x constituted by amino acids constituting an antibody medicine and an evaluation index p corresponding thereto. For this purpose, the reward function identification processing unit 13 acquires the amino acid received by the input unit 11 and creates the entire sequence x assumed by a combination thereof. In addition, the reward function identification processing unit 13 may receive sequence information indicating the entire sequence x via the input unit 11, or may acquire sequence information indicating the entire sequence x stored previously in the storage unit 17. Then, the reward function identification processing unit 13 acquires the corresponding evaluation index p from the storage unit 17. The amino acid herein is an example of an element.

In addition, in step S2, the reward function identification processing unit 13 encodes each piece of the acquired sequence information. For this purpose, one-hot encoding is used, for example.

In addition, in step S3, the reward function identification processing unit 13 solves $W^K$, $W^V$, $W^Q$, and $\beta 1_p$ using the evaluation index p of each encoded sequence information as teacher data.

Hereinafter, mathematical formulas (Mathematical formula 3) to (Mathematical formula 9) indicating the evaluation indexes p to $\beta 1_p$ are shown below.

[Mathematical formula 3]

$$K = W^K X = \left(W^k x^1, \ldots, W^k x^S\right) = \left(k^1, \ldots, k^S\right) \quad \text{... (Mathematical formula 3)}$$

[Mathematical formula 4]

$$V = W^V X = \left(W^V x^1, \ldots, W^V x^S\right) = \left(v^1, \ldots, v^S\right) \quad \text{... (Mathematical formula 4)}$$

[Mathematical formula 5]

$$Q = W^Q X = \left(W^Q x^1, \ldots, W^Q x^T\right) = \left(q^1, \ldots, q^T\right) \quad \text{... (Mathematical formula 5)}$$

[Mathematical formula 6]

$$\sigma_S^t = sc(k^S, q^t) \quad \text{... (Mathematical formula 6)}$$

[Mathematical formula 7]

$$\alpha^t = (\alpha_1^t, \ldots, \alpha_S^t) = softmax(\sigma^t) \quad \text{... (Mathematical formula 7)}$$

[Mathematical formula 8]

$$c^t = \sum_{s=1}^{S} \alpha_S^t v^S \in R^l \quad \text{... (Mathematical formula 8)}$$

[Mathematical formula 9]

$$p = \sum_{p=1}^{P} \beta_p^1 c_p^1 \in R \quad \text{... (Mathematical formula 9)}$$

In addition, in step S4, the reward function identification processing unit 13 specifies the reward function R and the attention $\alpha^t$ by using the result of step S3 and outputs the reward function R and the attention $\alpha^t$. This reward function R is specified using (Mathematical formula 10).

[Mathematical formula 10]

$$R(X) = \sum_{p=1}^{P} \beta_p^1 c_p^1 \in R \quad \text{... (Mathematical formula 10)}$$

In addition, in step S5, the reward function identification processing unit 13 learns the reward function R specified in step S4. For this purpose, the reward function identification processing unit 13 learns a prediction model for predicting characteristics such as affinity for an antigen, hydrophobicity, solubility, and immunogenicity as the reward function R. Herein, the prediction model can specify the relationship (attention) between amino acids (elements) in the sequence as the magnitude of contribution to the reward function R together with the characteristic value for each piece of the acquired sequence information.

Herein, attention specified by the prediction model will be described. FIG. 8 is a diagram for explaining attention in the present embodiment. In FIG. 8, $X_t$ indicates the sequence information, and can be indicated by a product of positions (elements) $x^t_n$ indicating each position of the sequence information. In addition, the degree of influence At in the sequence information $X_t$ can be represented by a product of the degree of influence $\alpha^t_{nm}$ in each adjacent pair. Herein, the sequence information $X_t$ is APRKN ···. These influence degrees indicate weights regarding importance of adjacent pairs during prediction processing using a prediction model. In particular, the antibody medicine has a steric structure, and thus amino acids at distant (non-adjacent) positions may affect each other.

Next, the degree of influence will be described. FIG. 9 is a diagram for explaining the degree of influence in the present embodiment. When the attention of the adjacent positions (elements) $x^t_{i+1}$ of the positions (elements) $x^t_i$ of the sequence information $X_t$ is averaged, the degree of influence $\alpha^{all}_{i,j}$ of the adjacent positions is specified from each amino acid of each position $x^{all}_{i,j}$. Herein, in FIG. 9, the thickness of the arrow between the respective elements indicates the strength of the degree of influence. Therefore, the left side of FIG. 9 illustrates that the degree of influence of the adjacent pair of A and any one of A, C, and D is strong. The right side of FIG. 9 indicates that the degree of influence of the adjacent pair of C and any one of C, D, E, and F is strong.

In addition, it can be treated that the contribution of the reward function R (x) also varies according to the degree of influence $\alpha^{all}_{i,j}$. For example, when the degree of influence $a^{all}_{i,j}$ is small, it can be treated that the contribution to the reward function R (x) is also small. Hereinafter, the description returns to the processing flow of FIG. 7.

In addition, in step S6, the attention analysis processing unit 14 aggregates the relationship (attention) between the amino acids in the entire acquired sequence information, and identifies adjacent pairs that weakly contribute to the reward function R at each position in the sequence information. Weak contribution means that the contribution is equal to or less than a threshold stored previously in the storage unit 17. In addition, "the contribution is weak " is an example, and the contribution of the adjacent pair may satisfy a predetermined condition.

In addition, in step S7, the sampling function identification processing unit 15 performs learning to obtain the sampling function proportional to the reward function R from the sampling function (F) that minimizes the loss function.

For this purpose, (Mathematical formula 11) is used. Details thereof will be described below. FIG. 10 is a flowchart illustrating sampling function of a learning processing in step S7 according to the present embodiment. In step S71 of FIG. 10, the sampling function identification processing unit 15 selects the learning data X for the sequence information.

[Mathematical formula 11]

$$P(s'|s) = \frac{F(s \to s')}{\sum_{s'' \in child(s)} F(s \to s'')} \quad \text{... (Mathematical formula 11)}$$

In addition, in step S72, the sampling function identification processing unit 15 selects an element s at a predetermined position from the acquired sequence information. In addition, in step S73, the sampling function identification processing unit 15 determines whether the contribution of the reward function R to the adjacent pair (s → s') including the selected element s is weak (equal to or less than a threshold value). As a result, in the case of being weak (Y), the process proceeds to step S75. In the case of not being weak (N), the process proceeds to step S74.

Herein, the strength of the contribution of the adjacent pair in step S73 will be described with reference to FIGS. 11 and 12. FIG. 11 is a diagram schematically illustrating strength of contribution of adjacent pairs in the present embodiment. In FIG. 11, A-C-D-F... in the vertical direction indicates an amino acid (symbol) that is an element of sequence information, and selecting this creates sequence information indicating an antibody medicine in the horizontal direction. In the figure, arrows connect adjacent pairs, and the thickness thereof indicates the magnitude (strength) of the relationship between the respective amino acids. Similarly to FIG. 9, FIG. 11 also illustrates that the degree of influence of the adjacent pair of A and any one of A, C, and D is strong. Then, FIG. 12 is a diagram schematically illustrating an evaluation index p of an adjacent pair according to one embodiment of the present invention. An evaluation index p for each of the elements s and s' to s''' will be identified. As described above, it can be determined from FIGS. 11 and 12 that an adjacent pair having a weak contribution to the reward function R is an adjacent pair having a small contribution to the reward function R.

Next, in step S74, the sampling function identification processing unit 15 calculates a loss function by (Mathematical formula 12).

[Mathematical formula 12]

$$L(s \to s') = \left( log \frac{\sum_{s' \in Parent(s)} F(s' \to s)}{\sum_{s'' \in child(s)} F(s \to s'')} \right)^2 \quad \text{... (Mathematical formula 12)}$$

$$F(x \to s_{end}) = R(x)$$

Herein, (Mathematical formula 12) represents an approximate expression of (Mathematical formula 13) representing the flow matching condition.

[Mathematical formula 13]

$$\sum_{s' \in Parent(s)} F(s' \to s) = \sum_{s'' \in child(s)} F(s \to s'') \quad \text{... (Mathematical formula 13)}$$

$$F(x \to s_{end}) = R(x)$$

In addition, in step S75, the sampling function identification processing unit 15 calculates a loss function by (Mathematical formula 14). As described above, different loss functions are used in steps S74 and S75.

[Mathematical formula 14]

$$L(s \rightarrow s') = a \sum_{s' \in child(s)} P(s \rightarrow s') log P(s \rightarrow s') + b|F(s \rightarrow s')| \quad \text{... (Mathematical formula 14)}$$

Herein, (Mathematical formula 14) has a regularization term that minimizes entropy and minimizes an F (s → s') value for an adjacent pair (s → s') in which the contribution of the reward function R is weak.

In addition, in step S76, the sampling function identification processing unit 15 determines whether the next position (element) is in the sequence information for which the loss function is to be calculated. In the case with the next position (Y), the process proceeds to step S72, and the subsequent steps are executed for the next position. In addition, in the case without the next position (N), the process proceeds to step S77.

In addition, in step S77, the sampling function identification processing unit 15 determines whether there is next learning data X to be processed in this flow. As a result, in the case with the data (Y), the process proceeds to step S71, and the subsequent steps are executed for the next learning data X. In addition, in the case without the data (N), this flow is ended, and the process proceeds to step S8 of step S7 in FIG. 7. This is the end of the description of step S7, that is, FIG. 10, and the description returns to FIG. 7.

Next, in step S8, the sequence creation processing unit 16 samples the symbol, that is, the set of elements based on (Mathematical formula 11) by the sampling function (F) obtained in step S7. As a result, sequence information is created.

As described above, the processing flow in the present embodiment ends, but the contents of the processing flow can be expressed as follows.

· An adjacent pair (s → s') satisfying a predetermined condition such as a weak contribution to the reward function R is an adjacent pair having a small contribution to the reward function R. Then, in the present embodiment, in order to ensure diversity of sequence information, a portion having a small contribution is utilized.

· In the minimization of the loss function, for an adjacent pair having a weak contribution to the reward function R, evaluation indexes for adjacent positions are (substantially) equal by entropy minimization. This equalization affects the achievement of the flow matching condition, but the value of F of the adjacent pair is reduced by the regularization term, and the influence is reduced to be minimized or the like.

· In an adjacent pair having a weak contribution to the reward function R, selection of adjacent positions by the sampling function is randomized, whereby a larger reward and a more diverse sequence can be sampled. Herein, the reward indicates a desired characteristic and a degree of efficacy, and the larger reward indicates the desired characteristic and reward are more easily obtained.

Note that this reward can be represented by a reward item indicating affinity, hydrophobicity, solubility, immunogenicity, and the like. Then, the learning data in step S71 may be sieved using the reward item. That is, the sampling function identification processing unit 15 selectively acquires learning data (antibody medicine of sequence information) in which the characteristic value of the reward item designated by the user or the like among the reward items satisfies a predetermined standard, and sieves out other items.

This is the end of the description of the present embodiment, but the present invention is not limited thereto. For example, elements are not limited to amino acids, and sequence information is not limited to amino acid sequences. For example, the present invention can also be applied to various chemical substances. Further, the present invention can also be applied to a neodymium magnet, a superconductor, a semiconductor, and the like formed by changing the arrangement of atoms. Further, the sequence information processing device 1 may also be implemented by a computer other than a server.

Reference Signs List

| | |
|---|---|
| 1 | sequence information processing device |
| 11 | input unit |
| 12 | output unit |
| 13 | reward function identification processing unit |
| 14 | attention analysis processing unit |
| 15 | sampling function identification processing unit |
| 16 | sequence creation processing unit |
| 17 | storage unit |
| 18 | sequence information |

| | |
|---|---|
| 19 | evaluation index |
| 101 | processing device |
| 102 | communication device |
| 103 | memory |
| 104 | sub-storage device |
| 2a to 2c | pharmaceutical companies |
| 20a to 20c | terminal device |
| 3 | Network |

## Claims

1. A sequence information processing device, processing sequence information represented by a sequence of a plurality of elements, the sequence information processing device comprising:

   an attention analysis processing unit that identifies, based on a reward function that quantifies a characteristic of the sequence information, an inter-element pair of sequence information in which contribution of the reward function to output satisfies a predetermined condition; and
   a sampling function identification processing unit that calculates a sampling function for creating the sequence information such that appearance frequencies of the plurality of elements are substantially equal in the identified inter-element pair.

2. The sequence information processing device according to claim 1, wherein the predetermined condition indicates whether or not contribution of the reward function to output is smaller than a predetermined threshold value.

3. The sequence information processing device according to claim 2, wherein the predetermined condition is that a loss function is minimized.

4. The sequence information processing device according to claim 1, wherein the sampling function identification processing unit selectively acquires learning data used for identification of the sampling function by using a reward item for the sequence information.

5. The sequence information processing device according to claim 1, wherein the sampling function identification processing unit uses different loss functions depending on whether or not the predetermined condition is satisfied in identification of the sampling function.

6. The sequence information processing device according to claim 5, wherein in a case where the predetermined condition is satisfied, a loss function having a regularization term that minimizes entropy is used.

7. The sequence information processing device according to claim 5, wherein in a case where the predetermined condition is not satisfied, an approximate expression of a calculation expression indicating a row matching condition is used.

8. The sequence information processing device according to claim 1, wherein the sequence information processing device further includes a reward function identification processing unit that identifies the reward function.

9. The sequence information processing device according to claim 1, comprising a sequence creation processing unit that creates new sequence information by using the sampling function.

10. The sequence information processing device according to any one of claims 1 to 9, wherein the element is a symbol indicating an amino acid, and the sequence information indicates an antibody medical product including a plurality of amino acids.

11. A sequence information processing device that processes sequence information represented by a sequence of a plurality of elements, the sequence information processing device comprising:

   an input unit that receives the plurality of elements as an input;
   an attention analysis processing unit that identifies an inter-element pair of sequence information in which contribution of a reward function to output satisfies a predetermined condition based on the reward function that quantifies a characteristic of the sequence information;
a sampling function identification processing unit that calculates a sampling function for creating the sequence information such that appearance frequencies of the plurality of elements are substantially equal in the identified inter-element pair; and
an output unit that outputs the created sequence information.

**12.** A sequence information processing method executed by a sequence information processing device represented by a sequence of a plurality of elements, the sequence information processing method implementing:

a process of identifying an inter-element pair of sequence information in which a contribution to an output of a reward function satisfies a predetermined condition based on the reward function that quantifies a characteristic of the sequence information; and
a process of calculating a sampling function for creating the sequence information such that appearance frequencies of the plurality of elements are substantially equal in the identified inter-element pair.

FIG. 1

X=APRKNVRWCTISQPEWFKCR

| SYM-BOL | AMINO ACID NAME | SYM-BOL | AMINO ACID NAME | SYM-BOL | AMINO ACID NAME | SYM-BOL | AMINO ACID NAME |
|---|---|---|---|---|---|---|---|
| A | ALANINE | G | GLYCINE | M | METHIONINE | S | SERINE |
| C | CYSTEINE | H | HISTIDINE | N | ASPARAGINE | T | THREONINE |
| D | ASPARTIC ACID | I | ISOLEUCINE | P | PROLINE | V | VALINE |
| E | GLUTAMATE | K | LYSINE | Q | GLUTAMINE | W | TRYPTO-PHAN |
| F | PHENYL-ALANINE | L | LEUCINE | R | ARGININE | Y | TYROSINE |

FIG. 2

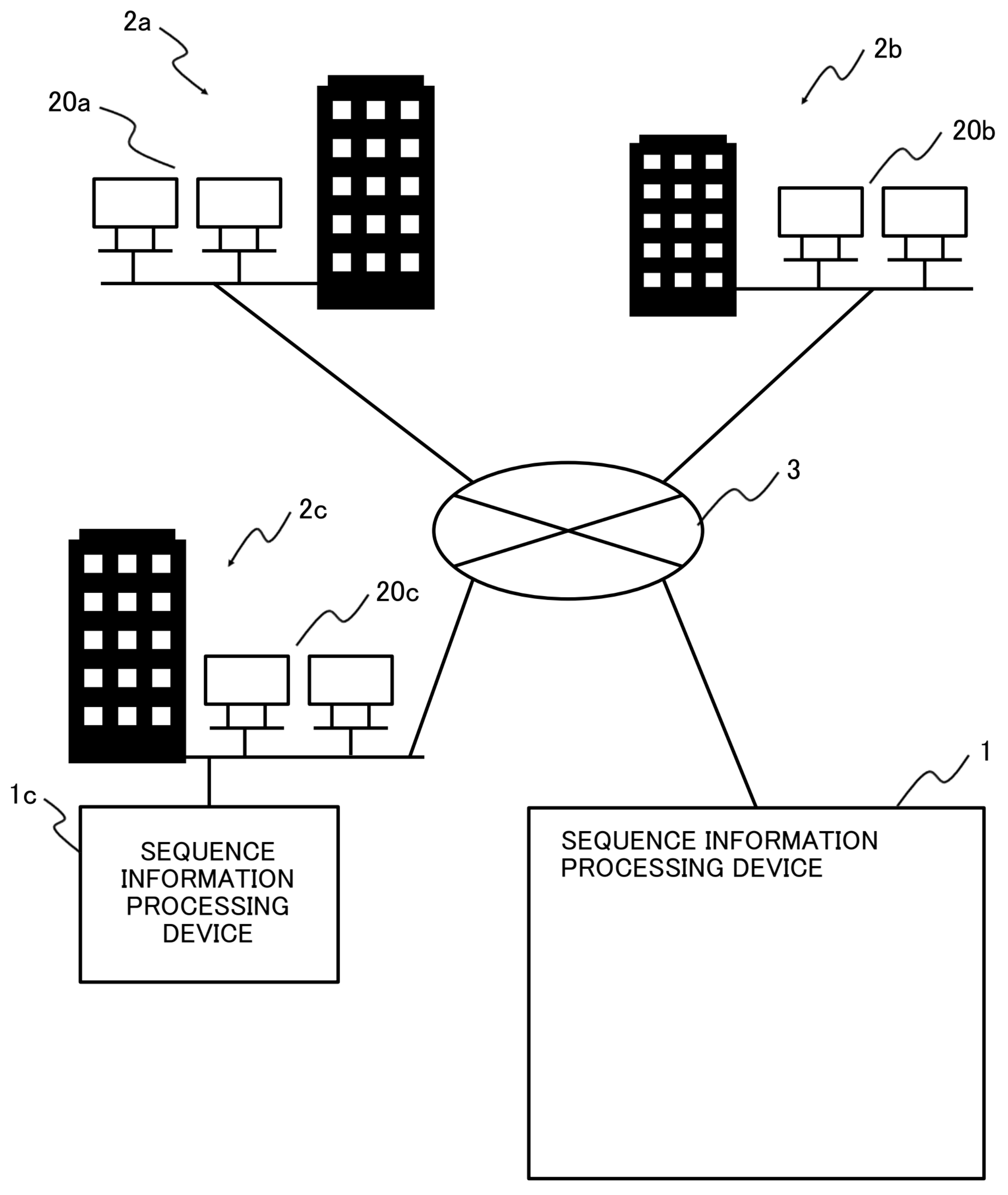
2a
20a
2b
20b
3
2c
20c
1c
SEQUENCE INFORMATION PROCESSING DEVICE
1
SEQUENCE INFORMATION PROCESSING DEVICE

FIG. 3

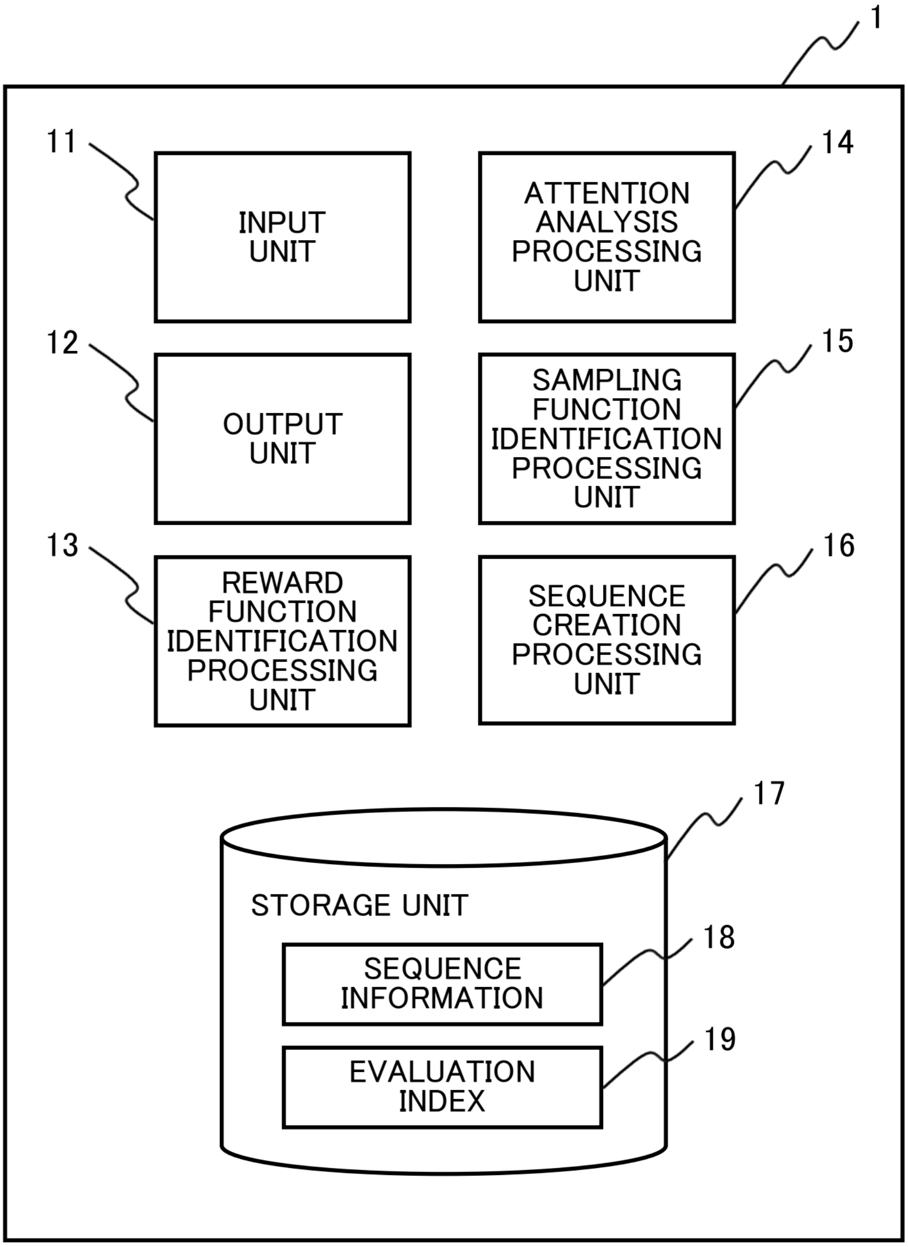
1
11
INPUT UNIT
14
ATTENTION ANALYSIS PROCESSING UNIT
12
OUTPUT UNIT
15
SAMPLING FUNCTION IDENTIFICATION PROCESSING UNIT
13
REWARD FUNCTION IDENTIFICATION PROCESSING UNIT
16
SEQUENCE CREATION PROCESSING UNIT
17
STORAGE UNIT
18
SEQUENCE INFORMATION
19
EVALUATION INDEX

FIG. 4

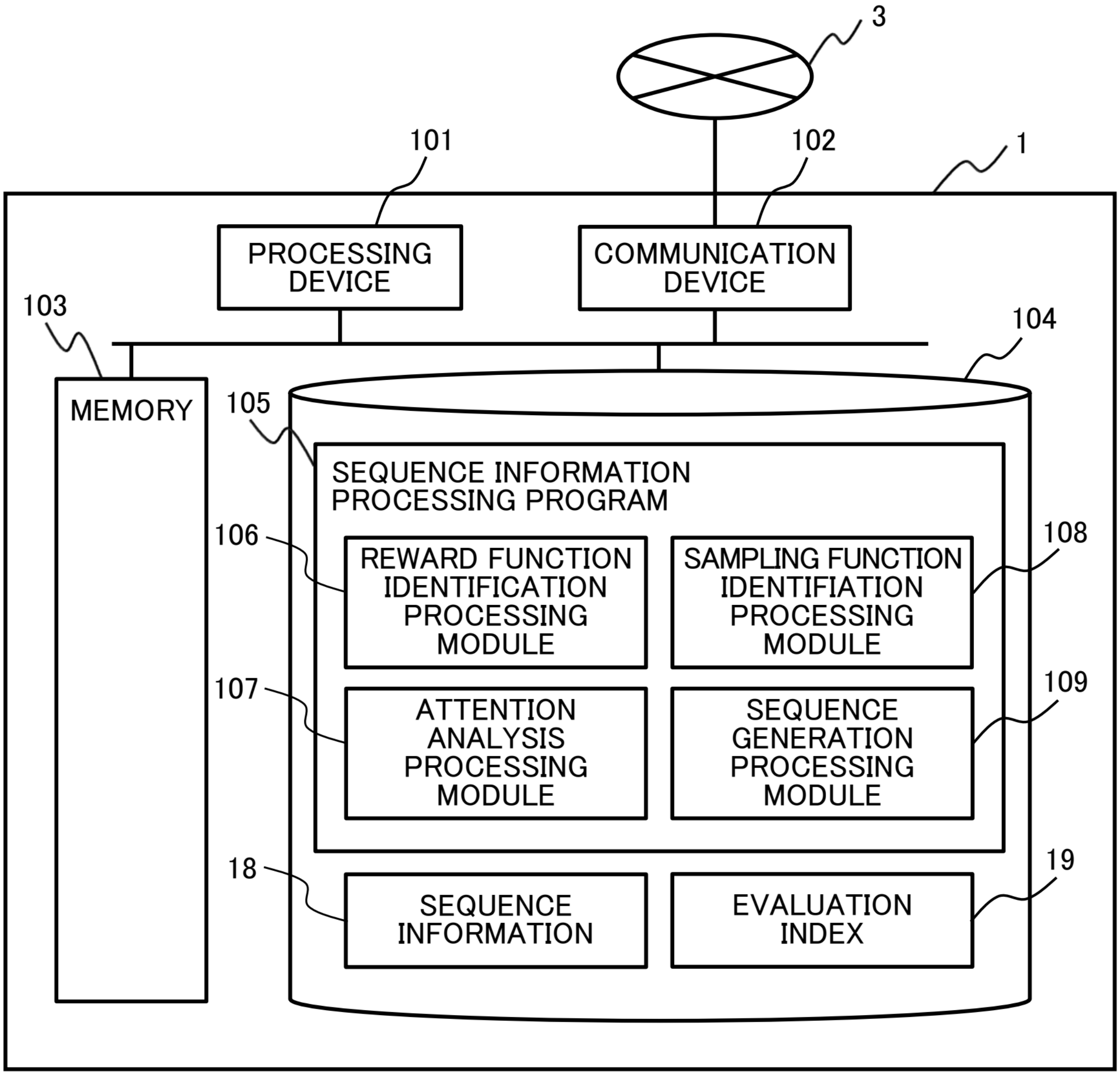
3
101
102
1
PROCESSING DEVICE
COMMUNICATION DEVICE
103
104
MEMORY
105
SEQUENCE INFORMATION PROCESSING PROGRAM
106
REWARD FUNCTION IDENTIFICATION PROCESSING MODULE
SAMPLING FUNCTION IDENTIFIATION PROCESSING MODULE
108
107
ATTENTION ANALYSIS PROCESSING MODULE
SEQUENCE GENERATION PROCESSING MODULE
109
18
SEQUENCE INFORMATION
EVALUATION INDEX
19

# *FIG. 5*

18

| NO | SYM-BOL 1 | SYM-BOL 2 | SYM-BOL 3 | SYM-BOL 3 | SYM-BOL 4 | SYM-BOL 5 | SYM-BOL 6 | SYM-BOL 7 | ... | SYM-BOL N |
|---|---|---|---|---|---|---|---|---|---|---|
| 1 | A | P | R | K | N | V | R | W | ... | R |
| 2 | A | P | R | L | N | V | R | W | ... | R |
| ... | ... | ... | ... | ... | ... | ... | ... | ... | ... | ... |
| M | A | X | L | R | Q | Y | H | K | ... | T |

# FIG. 6

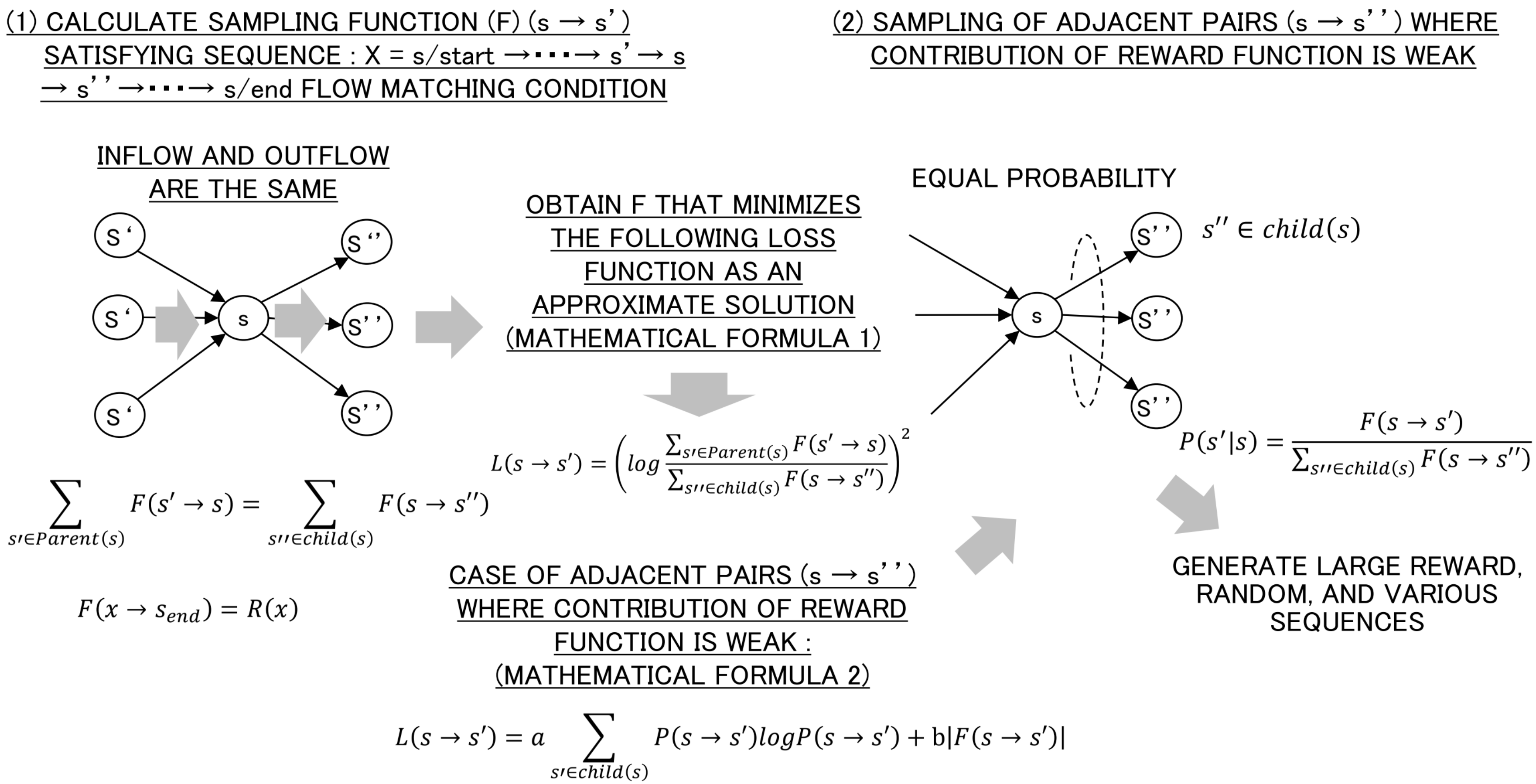
(1) CALCULATE SAMPLING FUNCTION (F) (s → s') SATISFYING SEQUENCE : X = s/start →・・・→ s' → s → s'' →・・・→ s/end FLOW MATCHING CONDITION
INFLOW AND OUTFLOW ARE THE SAME
s'
s
s''
$\sum_{s' \in Parent(s)} F(s' \to s) = \sum_{s'' \in child(s)} F(s \to s'')$
$F(x \to s_{end}) = R(x)$
OBTAIN F THAT MINIMIZES THE FOLLOWING LOSS FUNCTION AS AN APPROXIMATE SOLUTION (MATHEMATICAL FORMULA 1)
$L(s \to s') = \left( log \frac{\sum_{s' \in Parent(s)} F(s' \to s)}{\sum_{s'' \in child(s)} F(s \to s'')} \right)^2$
CASE OF ADJACENT PAIRS (s → s'') WHERE CONTRIBUTION OF REWARD FUNCTION IS WEAK : (MATHEMATICAL FORMULA 2)
$L(s \to s') = a \sum_{s' \in child(s)} P(s \to s') log P(s \to s') + b|F(s \to s')|$
(2) SAMPLING OF ADJACENT PAIRS (s → s'') WHERE CONTRIBUTION OF REWARD FUNCTION IS WEAK
EQUAL PROBABILITY
$s'' \in child(s)$
$P(s'|s) = \frac{F(s \to s')}{\sum_{s'' \in child(s)} F(s \to s'')}$
GENERATE LARGE REWARD, RANDOM, AND VARIOUS SEQUENCES

FIG. 7

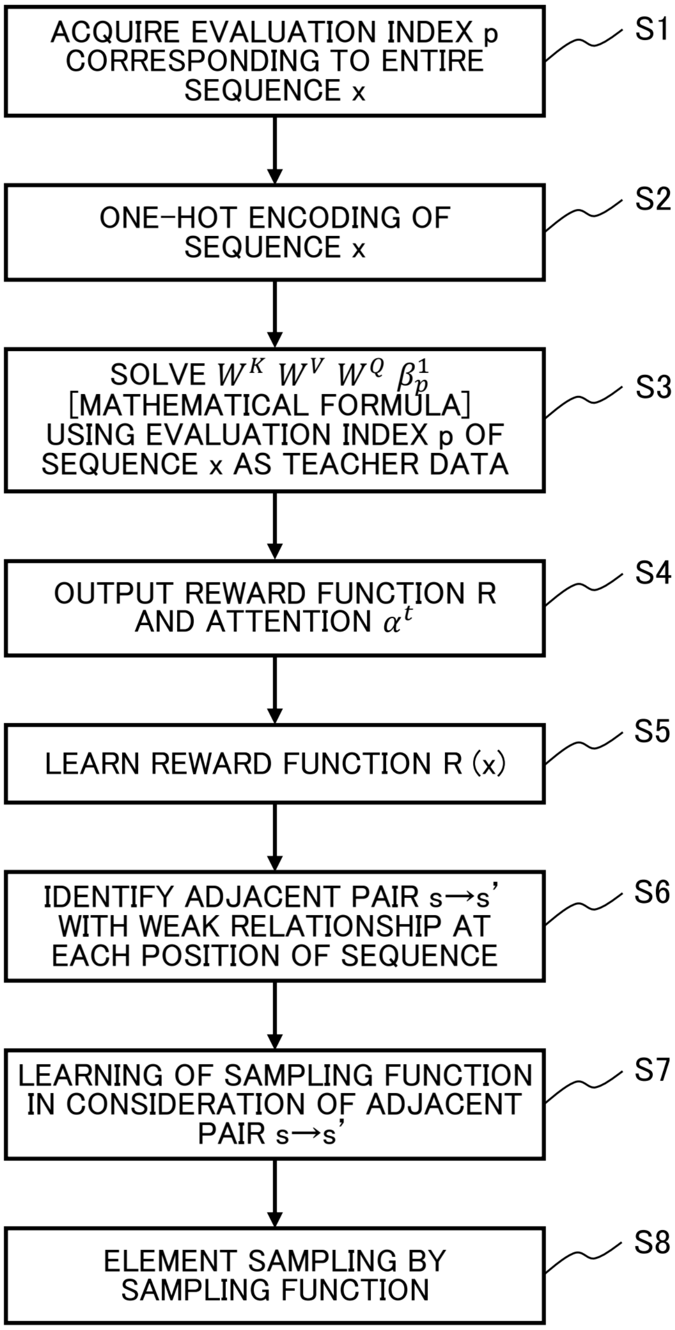
ACQUIRE EVALUATION INDEX p CORRESPONDING TO ENTIRE SEQUENCE x
S1
ONE-HOT ENCODING OF SEQUENCE x
S2
SOLVE $W^K$ $W^V$ $W^Q$ $\beta_p^1$ [MATHEMATICAL FORMULA] USING EVALUATION INDEX p OF SEQUENCE x AS TEACHER DATA
S3
OUTPUT REWARD FUNCTION R AND ATTENTION $\alpha^t$
S4
LEARN REWARD FUNCTION R (x)
S5
IDENTIFY ADJACENT PAIR s→s' WITH WEAK RELATIONSHIP AT EACH POSITION OF SEQUENCE
S6
LEARNING OF SAMPLING FUNCTION IN CONSIDERATION OF ADJACENT PAIR s→s'
S7
ELEMENT SAMPLING BY SAMPLING FUNCTION
S8

# FIG. 8

$$X_t = x_1^t x_2^t x_3^t x_4^t \cdots$$

$$A_t = (\alpha_{11}^t \alpha_{12}^t \alpha_{13}^t \alpha_{14}^t \cdots \alpha_{21}^t \alpha_{22}^t \alpha_{23}^t \alpha_{24}^t \cdots)$$

$X_t =$ APRKN···

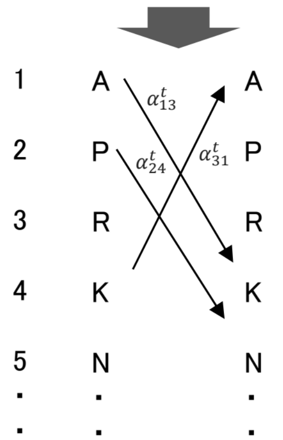


# FIG. 9

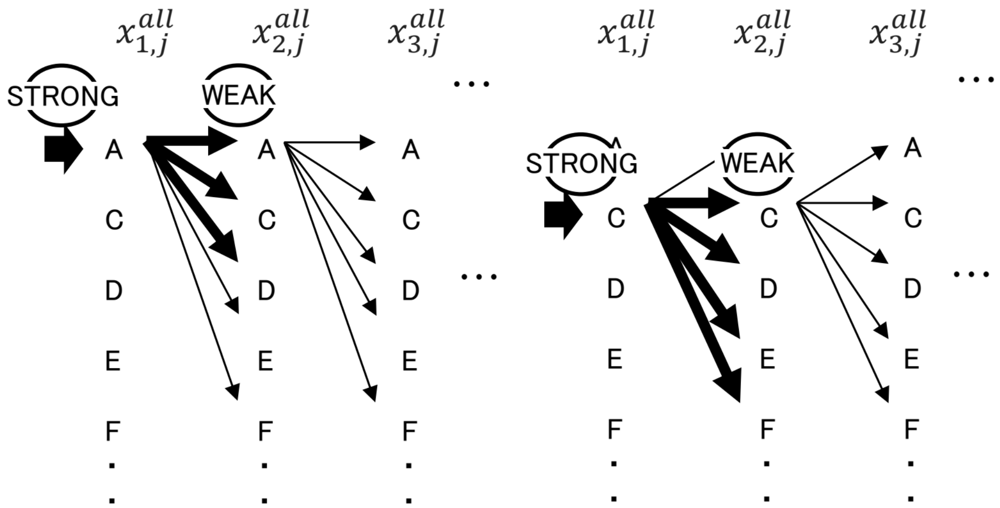

FIG. 10

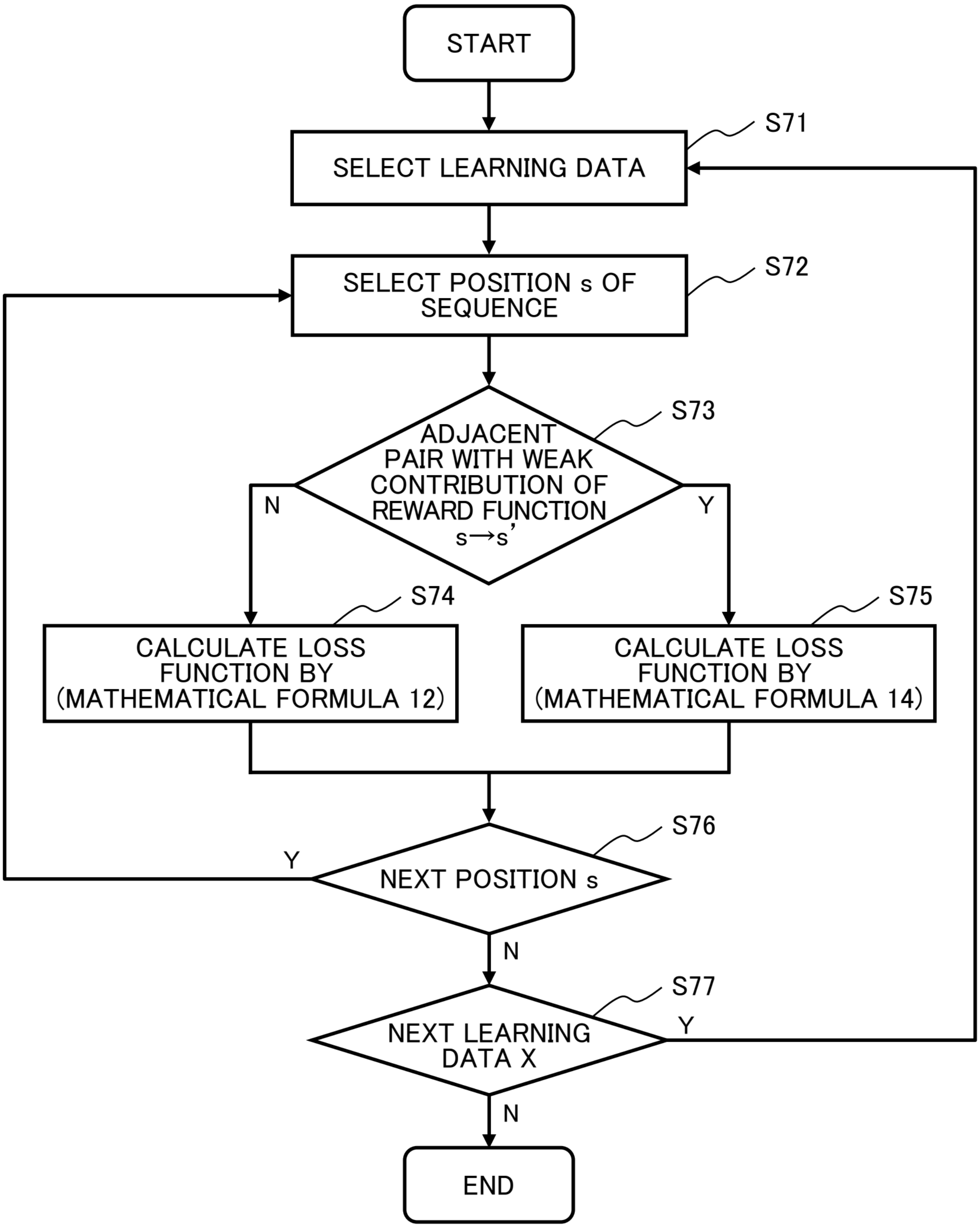
START
S71
SELECT LEARNING DATA
S72
SELECT POSITION s OF SEQUENCE
S73
ADJACENT PAIR WITH WEAK CONTRIBUTION OF REWARD FUNCTION s→s'
N
Y
S74
CALCULATE LOSS FUNCTION BY (MATHEMATICAL FORMULA 12)
S75
CALCULATE LOSS FUNCTION BY (MATHEMATICAL FORMULA 14)
S76
NEXT POSITION s
Y
N
S77
NEXT LEARNING DATA X
Y
N
END

FIG. 11

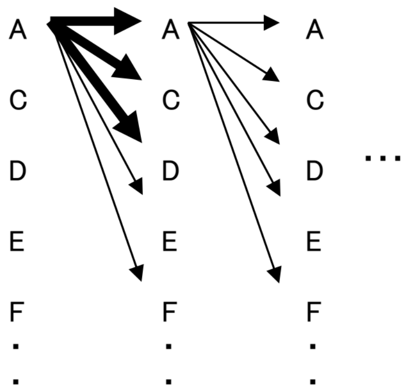


FIG. 12

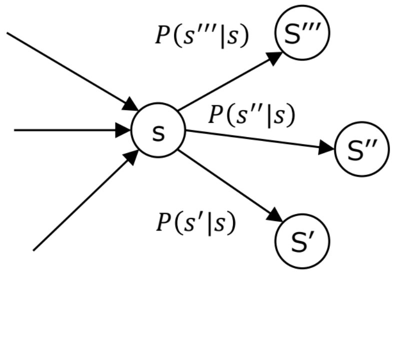

## INTERNATIONAL SEARCH REPORT

International application No.
**PCT/JP2024/004447**

**A. CLASSIFICATION OF SUBJECT MATTER**

***G16B 30/00***(2019.01)i; ***G16B 40/00***(2019.01)i
FI: G16B30/00; G16B40/00

According to International Patent Classification (IPC) or to both national classification and IPC

**B. FIELDS SEARCHED**

Minimum documentation searched (classification system followed by classification symbols)

G16B30/00; G16B40/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2022/216592 A1 (NEC LAB AMERICA INC.) 13 October 2022 (2022-10-13)<br>claims 1, 7, paragraphs [0006], [0033]-[0034], [0041]-[0044] | 1-4, 8-12<br>5–7 |
| A | US 2023/0085160 A1 (NEC LAB AMERICA INC.) 16 March 2023 (2023-03-16)<br>paragraph [0022] | 1-12 |
| A | WO 2022/056438 A1 (CHAN ZUCKERBERG BIOHUB INC.) 17 March 2022 (2022-03-17)<br>paragraph [0011] | 1-12 |
| A | WO 2020/246617 A1 (CHUGAI SEIYAKU KABUSHIKI KAISHA) 10 December 2020 (2020-12-10)<br>paragraphs [0157]-[0168] | 1-12 |
| A | JP 2020-77206 A (MIRACA RES INSTITUTE GK) 21 May 2020 (2020-05-21)<br>claims 4, 5 | 1-12 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 April 2024** | **23 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/004447**

| Patent document cited in search report | Publication date (day/month/year) | Patent family member(s) | Publication date (day/month/year) |
|---|---|---|---|
| WO 2022/216592 A1 | 13 October 2022 | US 2022/0327425 A1<br>DE 112022001980 T5<br>JP 2024-513241 A | |
| US 2023/0085160 A1 | 16 March 2023 | (Family: none) | |
| WO 2022/056438 A1 | 17 March 2022 | JP 2023-541193 A<br>US 2023/0326542 A1<br>EP 4211271 A1<br>AU 2021340886 A1<br>KR 10-2023-0074178 A | |
| WO 2020/246617 A1 | 10 December 2020 | US 2022/0253669 A1<br>EP 3982369 A1<br>CN 114008713 A | |
| JP 2020-77206 A | 21 May 2020 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2020077206 A